(19) Europäisches Patentamt
European Patent Office
Office européen des brevets

(11) Publication number: **0 285 141 B1**

(12) # EUROPEAN PATENT SPECIFICATION

(45) Date of publication of patent specification: **02.06.93**

(51) Int. Cl.5: **C07D 239/26**, C09K 19/34, G02F 1/13

(21) Application number: **88105193.2**

(22) Date of filing: **30.03.88**

The file contains technical information submitted after the application was filed and not included in this specification

(54) **Mesomorphic compound and liquid crystal composition containing same.**

(30) Priority: **31.03.87 JP 78001/87**
**07.03.88 JP 54500/87**

(43) Date of publication of application:
**05.10.88 Bulletin 88/40**

(45) Publication of the grant of the patent:
**02.06.93 Bulletin 93/22**

(84) Designated Contracting States:
**DE FR GB IT NL**

(56) References cited:
**WO-A-86/06401**
**WO-A-87/05012**
**WO-A-87/05018**

**PATENT ABSTRACTS OF JAPAN, vol. 11, no. 30 (C-400)[2477], 29th January 1987.**

(73) Proprietor: **CANON KABUSHIKI KAISHA**
**30-2, 3-chome, Shimomaruko, Ohta-ku**
**Tokyo(JP)**

(72) Inventor: **Nohira, Hiroyuki**
**51-5 Ohkuboryoke**
**Urawa-shi Saitama-ken(JP)**

Inventor: **Kamei, Masanao**
**Shinetsukagaku-ryo, 3-12-37 Isobe**
**Annaka-shi Gunma-ken(JP)**
Inventor: **Nakamura, Shinichi**
**Fujimi-so 2-1, 5-20-27 Sakawa**
**Urawa-shi Saitama-ken(JP)**
Inventor: **Iwaki, Takashi**
**Honatsugi-Inoue Bldg. 506 72-1 Onna,**
**Atsugi-shi**
**Kanagawa-ken(JP)**
Inventor: **Katagiri, Kazuharu**
**5-9-16-2 Ochiai**
**Tama-shi Tokyo(JP)**
Inventor: **Yamada, Yoko**
**Canon-ryo, 2-10-1 Asahi-cho**
**Atsugi-shi Kanagawa-ken(JP)**

(74) Representative: **Bühling, Gerhard, Dipl.-Chem. et al**
**Patentanwaltsbüro Tiedtke-Bühling-Kinne**
**Grupe-Pellmann-Grams-Struif-Winter-Roth**
**Bavariaring 4**
**W-8000 München 2 (DE)**

EP 0 285 141 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an optically active mesomorphic compound, a liquid crystal composition containing such a mesomorphic compound, and a liquid crystal device using such a liquid crystal composition.

There has been a well known type of liquid crystal devices using TN (twisted nematic) type liquid crystals as shown, for example, in "Voltage-Dependent Optical Activity of a Twisted Nematic Liquid Crystal" by M. Schadt and W. Helfrich, Applied Physics Letters, Vol. 18, No. 4 (Feb. 15, 1971), pp. 127-128. In this type of liquid crystal devices, the number of picture elements have been restricted, because there is a problem that a crosstalk phenomenon occurs when a device of a matrix electrode structure with a high density of picture elements is driven according to a multiplexing driving scheme. Further, their uses for display have been limited because of slow electric field response and poor visual angle characteristics.

As another type of liquid crystal device, there has been known one comprising a plurality of picture elements each connected to and subject to switching by a thin film transistor as a switching element. This type of liquid crystal device, however, is accompanied with problems such that production of thin film transistors on a substrate is very complicated, and production of a display device with a large picture area or screen is difficult.

In order to obviate the above-mentioned drawbacks of the conventional types of liquid crystal devices, Clark and Lagerwall have proposed the use of a liquid crystal device wherein a ferroelectric liquid crystal is disposed in a thin layer having a thickness less than 5 times that of the helical pitch thereof so that its helical structure is unwound to develop a bistability (e.g., U.S. Patent No. 4367924).

As the bistable liquid crystal, a ferroelectric crystal showing a chiral smectic C phase (SmC*) or H phase (SmH*) is generally used.

Such a ferroelectric liquid crystal has very rapid response speed on account of having spontaneous polarization, can also exhibit memorizable bistable state and further have excellent vision angle characteristic, and therefore it is suitable for a display of large capacity and large picture area.

Further, since a material used as a ferroelectric liquid crystal has an asymmetry, it can be used as a functional material to be used in the following types of optical devices in addition to the use as a ferroelectric liquid crystal material:

1) Those utilizing a cholesteric-nematic phase transition in a liquid crystal state (J.J. Wysoki, A. Adams and W. Haas: Phys. Rev. Lett., 20, 10204 (1968));
2) Those utilizing a guest-host effect of the White-Taylor type in a liquid crystal state (D.L. White and G.N. Taylor: J. Appl. Phys. 45, 4718 (1974)).

These optical devices are important as display devices and modulation devices, while the explanation of the individual systems is left to the respective references and omitted.

It has been understood that, in a method utilizing an electric field-responsive optical effect of a liquid crystal, it is desirable to introduce a polar group or a group providing a polar bond in a compound constituting the liquid crystal in order to enhance the responsive characteristic of the liquid crystal.

EP-A-0 225 195 discloses phenylpyrimidine-based compounds and liquid crystal compositions containing the same wherein the phenylpyrimidine-based compounds are represented by the following general formula:

$R^1\text{-}A\text{-}(X)_a\text{-}B\text{-}(Y)_b\text{-}C\text{-}(Z)_c\text{-}D\text{-}R^2$

wherein $R^1$ represents a straight or branched $C_{1-18}$ alkyl group, haloalkyl group, aralkyl group or haloaralkyl group with or without a chiral carbon atom, $R^2$ represents a straight or branched alkyl group, haloalkyl group, aralkyl group or haloaralkyl group with or without at least one chiral carbon atom, a, b, or c is one or more than one and a sum of a, b and c is two or more than two, A represents single bond, -O-, -COO-, -OCOO or OCO, B and C may be the same or different and selected from single bond, -COO-, -OCO-, -N=CH-,-CH=N-, $-CH_2O$, $-OCH_2-$ or -CH=CH-, D represents single bond, $-COOCH_2-$, -OCOO- or -OCO, X, Y and Z are 1,4-phenylene, 1,4-phenylene substituted with one or more than one halogen atom, cyano group or nitro group, 2,5-pyrimidine

( 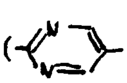 or 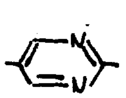 )

in which case at least one of X, Y or Z is 2,5-pyrimidine

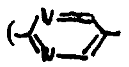 or 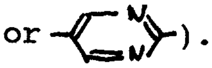

As a specific teaching with regard to technical action, this reference dicloses the use of (2S, 3S)-2-chloro-3-methylpentanoic acid as chiral optic carboxylic acid.

EP-A-0 191 860 discloses an optically active liquid crystal compound as shown in the following fomula:

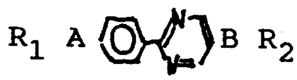

wherein A and B, respectively represent -, -O-.

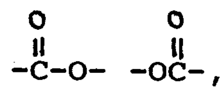

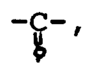

( - represents a direct bond), one of $R^1$ and $R^2$ shows direct chain alkyl groups and the other shows either the alkyl group holding asymmetric carbon atom or an alkoxy alkyl group holding asymmetric carbon atom and ether bonding; onto the asymmetric carbon atom one of the groups $-CH_3$, -CN, -Cl is added.

The aforementioned mesomorphic compounds having ferroelectric characteristics show as ferroelectric liquid crystals quite a rapid response speed due to their spontaneous polarization. However, it is still desirable to further improve the response speed. Since it is known that the response speed is proportional to the spontaneous polarization of the chiral liquid crystal, it is further desired to increase the spontaneous polarization in order to realize an improved response speed.

It is therefore the object of the present invention to provide a mesomorphic compound showing an impoved response speed and an increased spontaneous polarization, thus having enhanced electric field responsive characteristics in a liquid crystal state.

According to the invention this object is achieved by an optically active mesomorphic compound according to claim 1, a liquid crystal composition according to claim 14 and a liquid crystal device according to claim 18. The invention is advantageously developed by the measures mentioned in the subclaims.

According to the present invention, there is provided an optically active mesomorphic compound represented by the following formula (I):

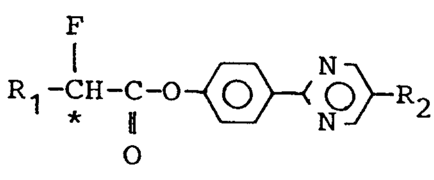 (I),

wherein $R_1$ and $R_2$ are respectively an alkyl group having 1 to 16 carbon atoms, and C* denotes an asymmetric carbon atom. Herein, the term "mesomorphic compound" is used to mean that the mesomorphic compound is not necessarily required to show a liquid crystal state by itself but it is sufficient that the mesomorphic compound is compatibly mixed with another mesomorphic compound to provide a liquid crystal composition showing a liquid crystal state.

According to the present invention, there are further provided a liquid crystal composition containing at least one species of the above mesomorphic compound as a constituent, and a liquid crystal device using the liquid crystal composition.

The mesomorphic compound represented by the above formula (I) may be synthesized from an optically active intermediate such as a 2-fluoroalkanoic acid of the following formula (II):

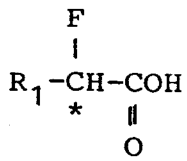

(II),

which in turn may be synthesized along the following scheme:

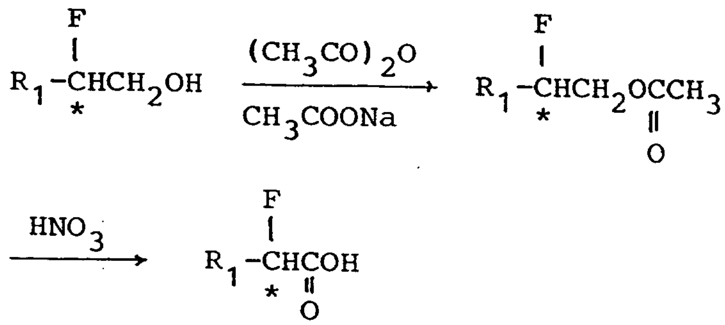


For example, the mesomorphic compound represented by the formula (I) nay be produced from the 2-fluoroalkanoic acid along the following scheme:

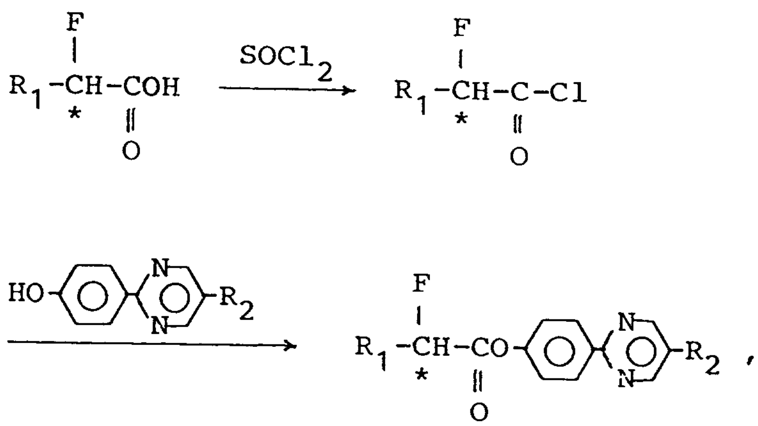


wherein $R_1$ and $R_2$ are the same as defined above.

The optically active mesomorphic compound according to the present invention represented by the formula (I) can have a wide variety of $R_1$ by changing the number of carbon atoms in the alkane moiety in the starting 2-fluoroalkanoic acid but those having an alkyl group $R_1$ of 1 - 16 are provided by the present invention.

Hereinbelow, specific examples of the mesomorphic compound (I) according to the present invention are enumerated hereinbelow:

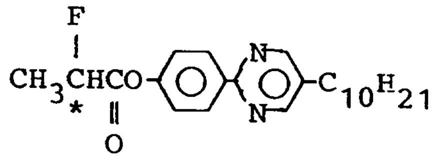

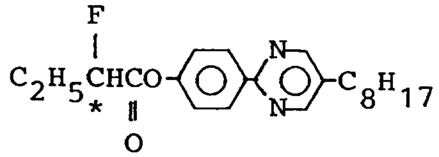

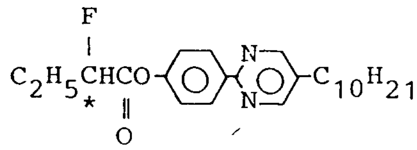

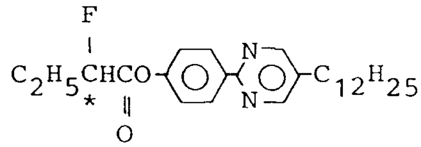

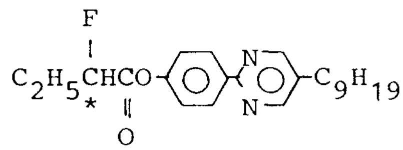

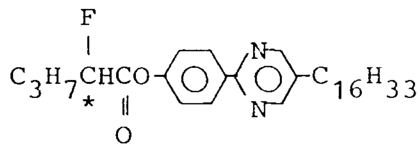

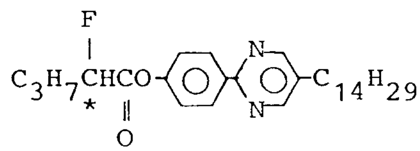

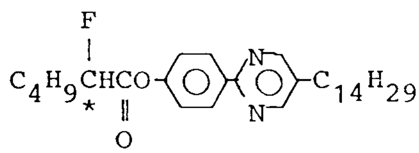

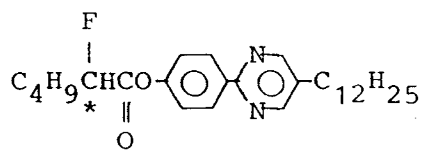

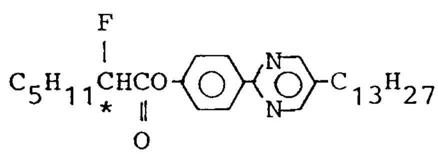

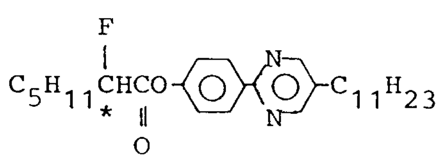

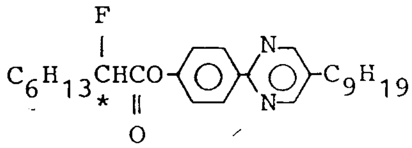

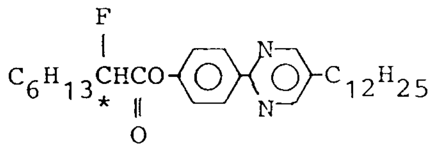

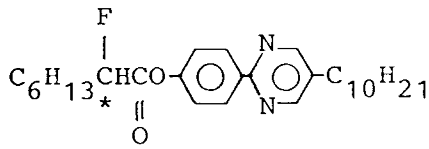

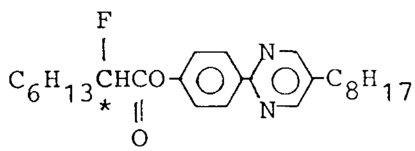

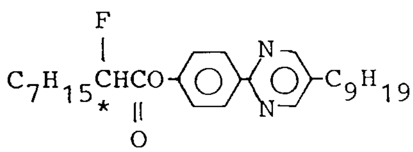

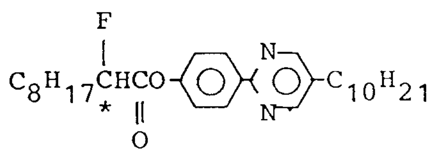

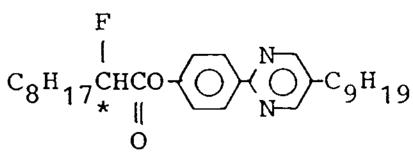

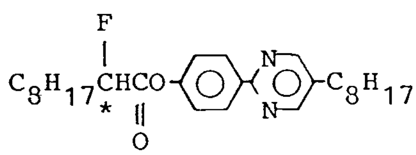

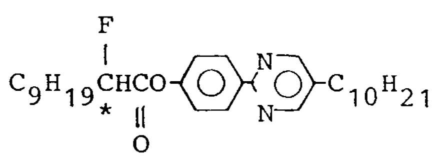

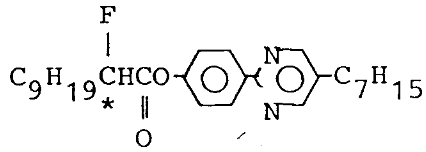

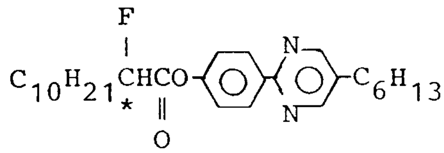

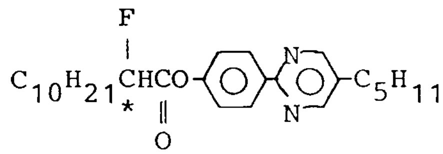

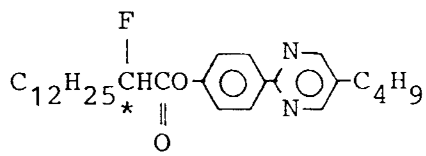

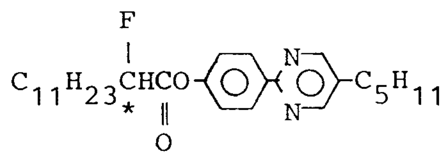

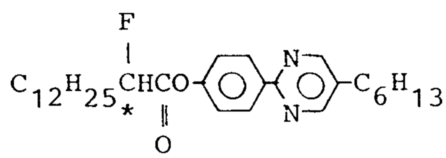

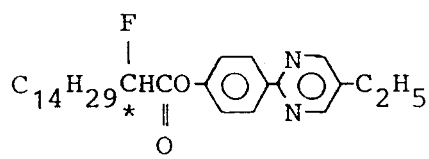

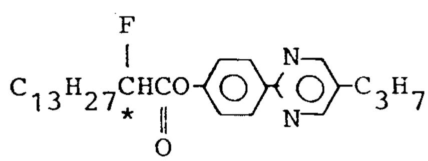

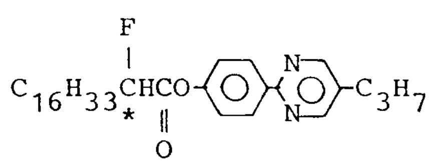

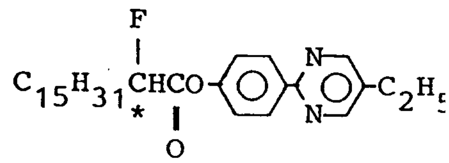

The liquid crystal composition according to the present invention contains at least one species of the mesomorphic compound represented by the formula (I). For example, the mesomorphic compound represented by the formula (I) may be mixed with a ferroelectric liquid crystal selected from those of the formulas <1> - <13> shown below to increase the spontaneous polarization and increase the response speed. In this case, it is preferred to use the mesomorphic compound represent by the formula (I) in an amount constituting 0.1 - 99 wt. %, particularly 1 - 90 wt. % of the resulting liquid crystal composition.

<1>

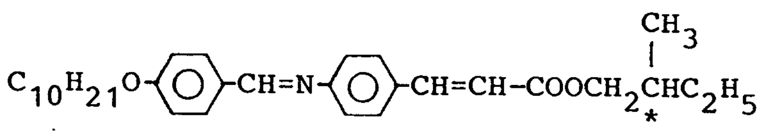

p-decyloxybenzylidene-p'-amino-2-methylbutylcinnamate

(DOBAMBC)

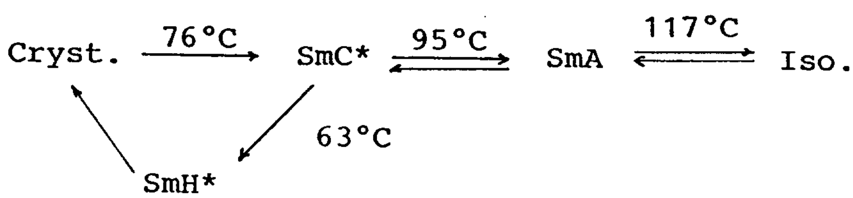

<2>

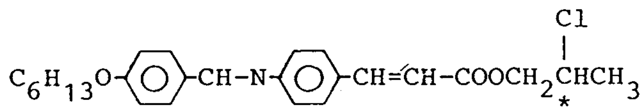

p-hexyloxybenzylidene-p'-amino-2-chloropropylcinnamate (HOBACPC)

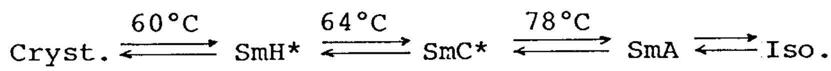


<3>

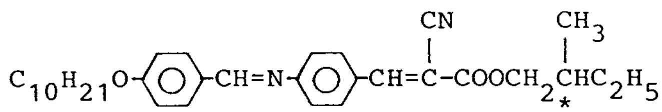

p-decyloxybenzylidene-p'-amino-2-methylbutyl-α-cyanocinnamate (DOBAMBCC)

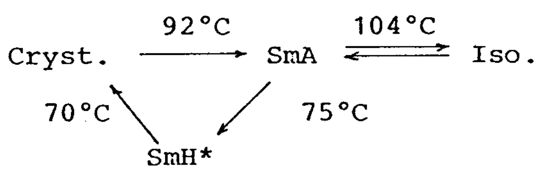


<4>

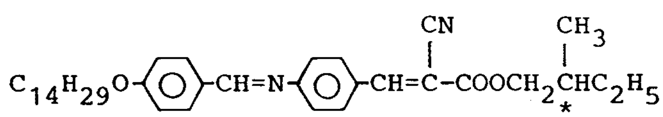

p-tetradecyloxybenzylidene-p'-amino-2-methylbutyl-α-cyanocinnamate (TDOBAMBCC)

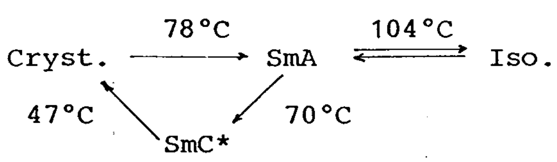

<5>

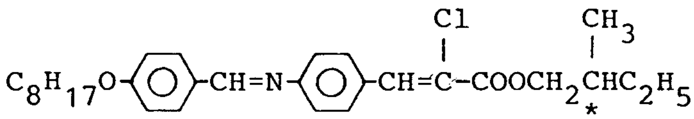

p-octyloxybenzylidene-p'-amino-2-methylbutyl-α-chlorocinnamate (OOBAMBCC)

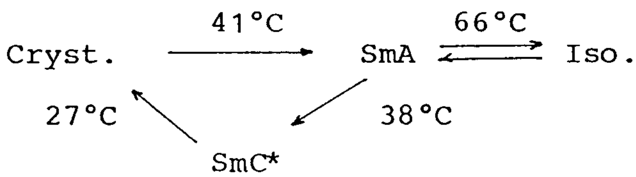


<6>

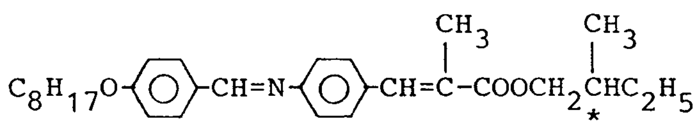

p-octyloxybenzylidene-p'-amino-2-methylbutyl-α-methylcinnamate

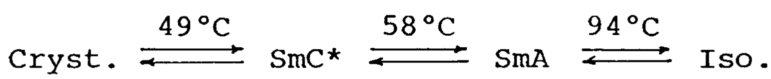


<7>

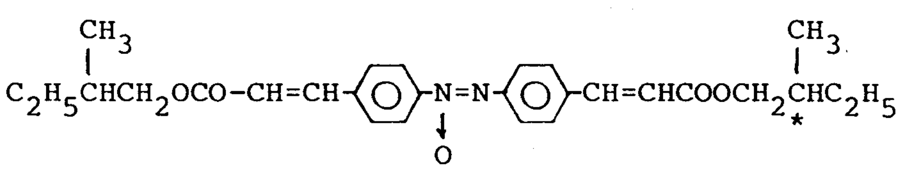

4,4'-azoxycinnamic acid-bis(2-methylbutyl)ester

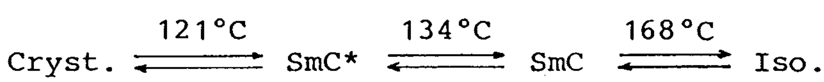

<8>

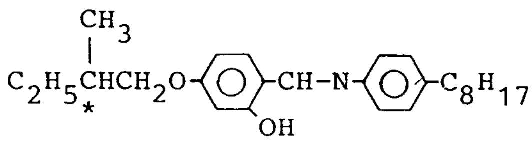

4-O-(2-methylbutyl)resorcylidene-4'-octylaniline

Cryst. $\overset{28^{\circ}C}{\rightleftarrows}$ SmC* $\overset{55^{\circ}C}{\rightleftarrows}$ SmA $\overset{62^{\circ}C}{\rightleftarrows}$ Iso.

<9>

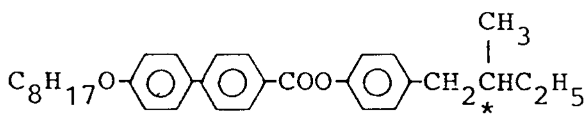

4-(2'-methylbutyl)phenyl-4'-octyloxybiphenyl-4-carboxylate

Cryst. $\overset{78^{\circ}C}{\rightleftarrows}$ Sm3 $\overset{80^{\circ}C}{\rightleftarrows}$ SmC* $\overset{128.3^{\circ}C}{\rightleftarrows}$ SmA $\overset{171.0^{\circ}C}{\rightleftarrows}$ Ch. $\overset{174.2^{\circ}C}{\rightleftarrows}$ Iso.

<10>

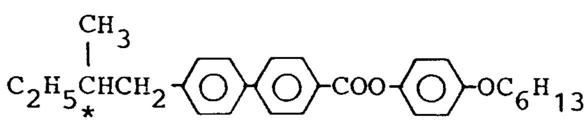

4-hexyloxyphenyl-4-(2"-methylbutyl)biphenyl-4'-carboxylate

Cryst. $\overset{68.8^{\circ}C}{\rightleftarrows}$ SmC* $\overset{80.2^{\circ}C}{\rightleftarrows}$ Ch. $\overset{163.5^{\circ}C}{\rightleftarrows}$ Iso.

<11>

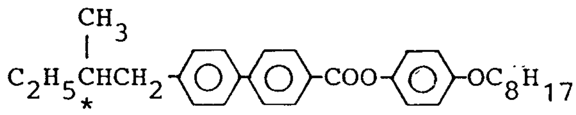

4-octyloxyphenyl-4-(2"-methylbutyl)biphenyl-4'-carboxylate

Cryst. ⇄ (76°C) SmC* ⇄ (88.6°C) Ch. ⇄ (155.4°C) Iso.

<12>

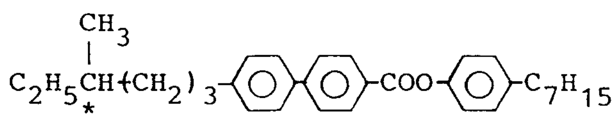

4-heptylphenyl-4-(4"-methylhexyl)biphenyl-4'-carboxylate

Cryst. ⇄ (91.5°C) SmC* ⇄ (93°C) SmA ⇄ (112°C) Ch. ⇄ (131°C) Iso.

<13>

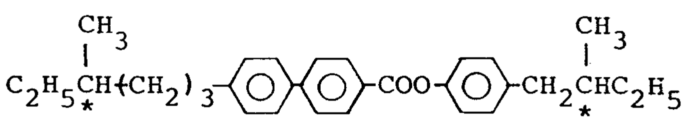

4-(2"-methylbutyl)phenyl-4-(4"-methylhexyl)biphenyl-4'-carboxylate

Cryst. → (83.4°C) Ch. → (114°C) Iso.

Ch. → (81.0°C) SmA

SmA → (74.3°C) SmC*

The mesomorphic compound represented by the formula (I) may also be mixed with a smectic liquid crystal such as those of the formulas <14> - <18> below which per se are not chiral to provide a composition which may be used as a ferroelectric liquid crystal. In this case, the mesomorphic compound represented by the formula (I) may preferably be used in an amount of 0.1 - 99 wt. %, particularly 1 - 90 wt. %. The resultant composition may be provided with an increased spontaneous polarization corresponding to

the content of the mesomorphic compound according to the present invention.

<14>

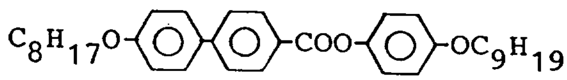

(4-nonyloxyphenyl)-4'-octyloxybiphenyl-4-carboxylate

Cryst. $\underset{74°C}{\overset{107°C}{\rightleftarrows}}$ SmB $\overset{117°C}{\longleftrightarrow}$ SmC $\overset{160°C}{\longleftrightarrow}$ SmA $\overset{195°C}{\longleftrightarrow}$ Iso.

<15>

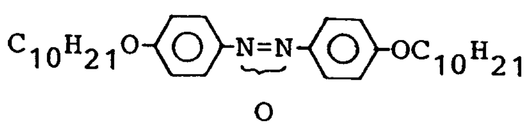

4,4'-decyloxyazoxybenzene

Cryst. $\overset{77°C}{\longrightarrow}$ SmC $\overset{120°C}{\longleftrightarrow}$ N $\overset{123°C}{\longleftrightarrow}$ Iso.

<16>

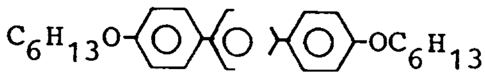

2-(4'-hexyloxyphenyl)-5-(4-hexyloxyphenyl)-pyrimidine

Cryst. $\xrightarrow{120°C}$ SmC $\xleftrightarrow{189°C}$ SmA $\xleftrightarrow{216°C}$ Iso.

<17>

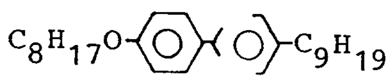

2-(4'-octyloxyphenyl)-5-nonylpyrimidine

Cryst. $\xrightarrow{33°C}$ SmC $\xleftrightarrow{60°C}$ SmA $\xleftrightarrow{75°C}$ Iso.

<18>

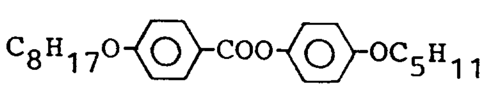

4'-pentyloxyphenyl-4-octylazoxybenzoate

Cryst. $\xrightarrow{58°C}$ SmC $\xrightarrow{64°C}$ SmA $\xrightarrow{66°C}$ N $\xrightarrow{85°C}$ Iso.

Herein, the symbols respectively denote the following phases:

| | |
|---|---|
| Cryst. | : crystal phase |
| SmA | : smectic A phase |
| SmB | : smectic B phase |
| SmC | : smectic C phase |
| N | : nematic phase |
| Iso. | : isotropic phase |

Further, the mesomorphic compound represented by the formula (I) is effective to prevent occurrence of reverse domain in a TN-type cell when it is added to a nematic liquid crystal. In this case, the mesomorphic compound represented by the formula (I) may preferably be used in an amount of 0.01 - 50 wt.% of the resultant liquid crystal composition.

Further, it is possible to add the mesomorphic compound according to the present invention to a nematic liquid crystal or a chiral nematic liquid crystal to provide a chiral nematic liquid crystal composition

which may be effectively used in a phase-transition type liquid crystal device or a White-Taylor type guest host liquid crystal device. In this case, the mesomorphic compound represented by the formula (I) may preferably be used in an amount of 0.01 - 80 wt.% of the resultant liquid crystal composition.

Hereinbelow, the present invention will be explained in more detail with reference to the Examples.

## Example 1

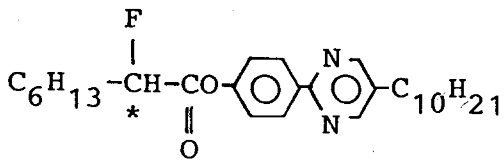

5-n-decyl-2-[4-(2-fluorooctanoyloxy)phenyl]-pyrimidine represented by the above formula was prepared along the following scheme;

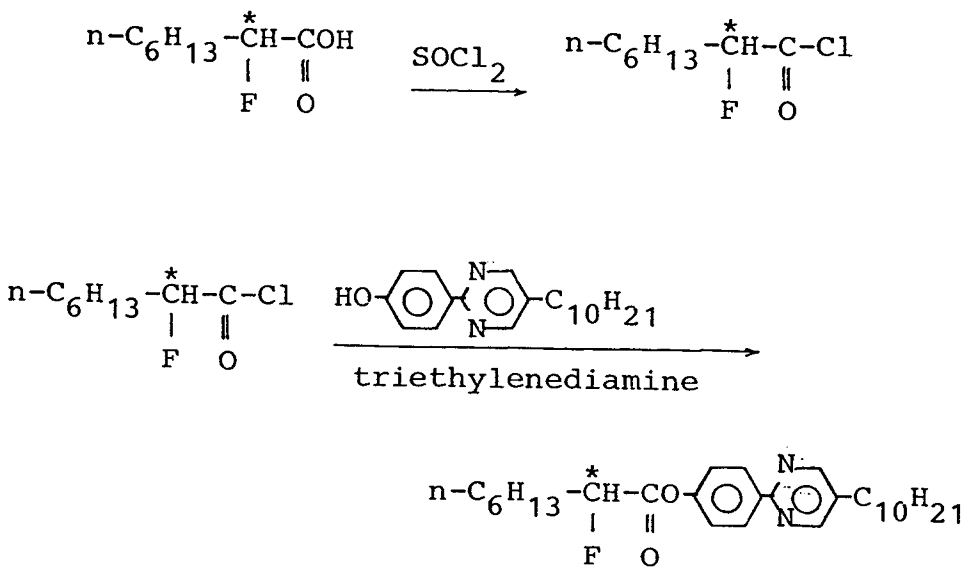


More specifically, 0.24 mg (1.5 mmol) of 2-fluorooctanoic acid was added to 2 ml of thionyl chloride and the mixture was refluxed at 90 °C for 2 hours. Then, the excess of thionyl chloride was distilled off, and a preliminarily prepared solution of 0.34 g (3.0 mmol) of triethylenediamine and 0.47 g (1.5 mmol) of 5-n-decyl-2-(p-hydroxyphenyl)pyrimidine in 5 ml of dry benzene was quickly added thereto, followed by 2 hours of stirring at 50 °C. Thereafter, 0.06 g (1.5 mmol) of sodium hydride (60 %) was added, and the mixture was further refluxed for 2 hours at 90 °C. After the reaction, 2N-hydrochloric acid was added to the system, which was then subjected to extraction with benzene. The extract was dried, subjected to distillation of the solvent and purified by column chromatography with benzene to obtain 0.44 g (0.96 mmol) of 5-n-decyl-2-[4-(2-fluorooctanoyloxy)phenyl]pyrimidine. Yield: 64 %

$$[\alpha]_D^{26.0} \quad +3.14° \quad (c=1.018,\ CH_2Cl_2)$$

$$[\alpha]_{435}^{24.8} \quad +12.6° \quad (c=1.018,\ CH_2Cl_2),$$

wherein c denotes a concentration expressed in terms of g/c.c.

Examples 2 - 7

Mesomorphic compounds of Examples 2 - 7 shown in the following Table 1 were prepared similarly as in Example 1.

The optical rotations and phase transition temperatures of the thus prepared mesomorphic compounds of Examples 1 - 7 are also shown in the following Table 1. In the table, Cryst. denotes a crystalline state; Iso., isotropic liquid; Ch., cholesteric phase; SmA, smectic A phase; SmC*, chiral smectic C phase; $S_C$*, a chiral smectic phase (un-identified); and $S_3$, a smectic phase (un-identified).

Table 1

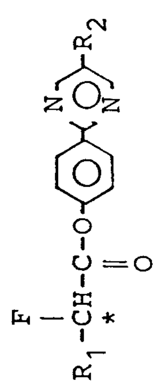

| Example | $R_1$ | $R_2$ | Optical rotation ($[\alpha]_D$) | Phase transition temperature (°C) |
|---|---|---|---|---|
| 1 | $n-C_6H_{13}-$ | $-C_{10}H_{21}$ | +3.14° (26°C) c=1.018, $CH_2Cl_2$ | Cryst. →(57) Iso.; Iso. →(45) Sc* →(32) $S_3$ →(27) Cryst. |
| 2 | $n-C_8H_{17}-$ | $-C_8H_{17}$ | +2.55° (17.6°C) c=0.71, $CH_2Cl_2$ | Cryst. →(55) Iso; Iso →(40) Cryst. |
| 3 | $n-C_8H_{17}-$ | $-C_9H_{19}$ | +2.54° (27.6°C) c=0.71, $CH_2Cl_2$ | Cryst. →(64) Iso; Iso →(46) $S_A$ →(42) Cryst. |
| 4 | $n-C_8H_{17}-$ | $-C_{10}H_{21}$ | +2.02° (27.2°C) c=0.69, $CH_2Cl_2$ | Cryst. →(59) Iso.; Iso. →(48) Ch. →(46) $S_A$ →(43) Sc* →(33) Cryst. |

..cont.

Table 1 (cont.)

| | | | | |
|---|---|---|---|---|
| 5 | $n-C_6H_{13}-$ | $-C_8H_{17}$ | +4.6° (24.0°C) c=0.914, $CH_2Cl_2$ | Cryst. —56→ Iso. —38→ $S_A$ —30→ Cryst. |
| 6 | $n-C_6H_{13}-$ | $-C_9H_{19}$ | +3.29° (24.8°C) c=0.98, $CH_2Cl_2$ | Cryst. —53→ Iso. —46→ $S_A$ —33→ Cryst. |
| 7 | $n-C_6H_{13}-$ | $-C_{12}H_{25}$ | +3.24° (18.4°C) c=0.93, $CH_2Cl_2$ | Cryst. —62→ Iso. —52→ Sc* —47→ Cryst. |

Example 8

A liquid crystal composition A shown below was prepared by using the mesomorphic compound of Example 1 above. The phase transition temperatures and spontaneous polarizations of the liquid crystal composition A are shown below.

<Liquid Crystal Composition A>

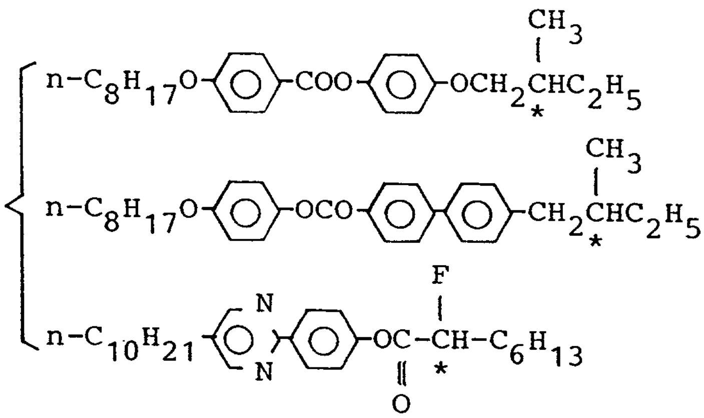

72.0 wt.%

18.0 wt.%

10.0 wt.%

Phase transition temperature (°C)

$$\text{Cryst.} \underset{10}{\overset{17}{\longleftrightarrow}} \text{Sc}^* \underset{44}{\overset{45}{\longleftrightarrow}} S_A \underset{60}{\overset{61}{\longleftrightarrow}} \text{Ch.} \underset{70}{\overset{70}{\longleftrightarrow}} \text{Iso}$$

Spontaneous polarization (nC/cm$^2$)

| Temp. (°C) | L. C. Composition A. |
|---|---|
| 40 | 4.3 |
| 30 | 7.7 |

Separately, two 0.7 mm-thick glass plates were provided and respectively coated with an ITO film to form an electrode for voltage application, which was further coated with an insulating layer of vapor-deposited $SiO_2$. On the insulating layer, a 0.2 %-solution of silane coupling agent in isopropyl alcohol was applied by spinner coating at a speed of 2000 rpm for 15 second and subjected to hot curing treatment at 120°C for 20 minutes.

Further, each glass plate provided with an ITO film and treated in the above described manner was coated with a 2 %-solution of polyimide resin precursor in dimethylacetoamide by a spinner coater rotating at 2000 rpm for 15 seconds. Thereafter, the coating film was subjected to heat curing at 300°C for 60 min. to obtain about 70 nm (700 Å) thick film. The coating film was rubbed with acetate fiber-planted cloth. The thus treated two glass plates were washed with isopropyl alcohol. After alumina beads with an average particle size of 1.5 µm were dispersed on one of the glass plates, the two glass plates were applied to each other with a bonding sealing agent so that their rubbed directions were parallel to each other and heated at 100°C for 60 minutes to form a blank cell. The cell gap was found to be about 2 µm as measured by a Berek compensator.

The cell thus prepared was filled under vacuum with the above-prepared liquid crystal composition A, heated into an isotropic phase, and gradually cooled at a rate of 0.5°C/hr to obtain a ferroelectric liquid crystal device.

Then, the optical response time (time from voltage application until the transmittance change reaches 90 % of the maximum) was measured for the device under the application of a peak-to-peak voltage of 30 V in combination with right-angle cross-nicol polarizers.

Response time (msec)

| Temp. (°C) | L.C. Composition A |
|---|---|
| 40 | 0.06 |
| 30 | 0.13 |

Example 9

Optically active 2-fluorobutanoic acid-p-(5-dodecyl-2-pyrimidyl)pheny-ester was prepared according to the following reaction scheme:

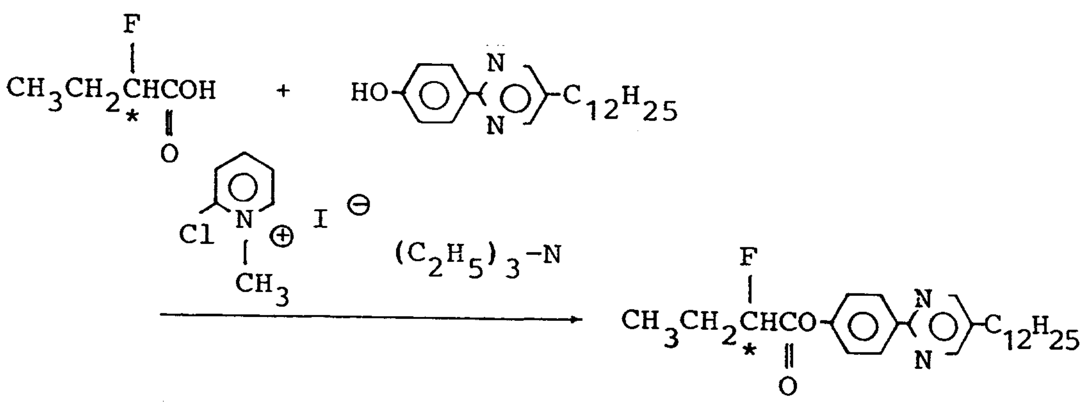


More specifically, into a solution of 0.34 g (1.0 mmol) of p-(5-dodecyl-2-pyrimidyl)phenol and 0.22 g (2.0 mmol) of triethylamine in dry dichloromethane, a solution of 0.11 g (1.0 mmol) of (+)-2-fluorobutanoic acid in dry dichloromethane and 0.31 g (1.2 mmol) of 2-chloro-1-methylpyridinium iodide were added, and the resultant mixture was refluxed under heating for 1 hour in nitrogen atmosphere. After the reaction was completed, dichloroethane was distilled off, and the product was purified by column chromatography (eluent: dichloromethane) and recrystallization from hexane. As a result, 0.11 g (0.27 mmol) of 2-fluorobutanoic acid-p-(5-dodecyl-2-pyrimidyl)phenyl-ester was obtained. The yield was 27 %.

$[\alpha]_D^{27}$ -1.22 (c = 0.82, dichloromethane)

Example 10

Optically active 2-fluoropropanoic acid-p-(5-decyl-2-pyrimidyl)phenyl-ester was prepared along the following scheme.

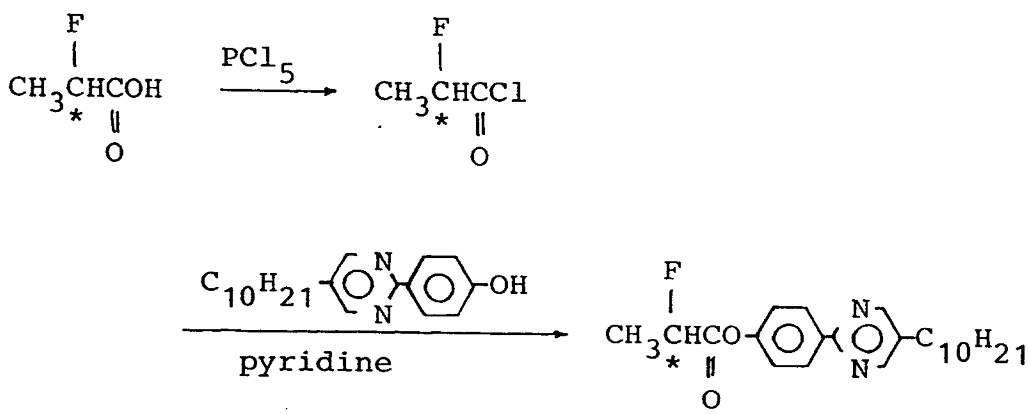

Thus, 1.6 g (174 mmol) of 2-fluoropropanoic acid was dissolved in 10 ml of benzene, to which 177 mmol of phosphorus pentoxide was added little by little in 20 minutes under stirring. Then, the mixture was raised in temperature and refluxed under heating for four hours to obtain an acid chloride.

5.4 g (173 mmol) of p-(5-decyl-2-pyrimidyl)phenol and 1.4 g (177 mmol) of pyridine were dissolved in 30 ml of benzene, and a benzene solution of the above 2-fluoropropanoic acid chloride was added dropwise thereto at 5 ° C in 15 minutes. Then, the mixture was stirred overnight at room temperature, and after the completion of the reaction, the reaction solution was poured into ice water. Then, the system was acidified with the addition of 6N-HCl and subjected to extraction with benzene. The extract benzene solution was washed with water and saline water, dried with anhydrous magnesium sulfate, and the solvent was distilled to leave 3.2 g of a crude product. The product was purified by column chromatography (eluent: hexane/acetone = 10/1) to obtain 330 mg of 2-fluoropropanoic acid-p-(5-decyl-2-pyrimidyl)phenyl-ester Yield: 5.0 %.

Examples 11 - 13

Example 9 was repeated by using p-(5-octyl-2-pyrimidyl)phenol, (5-nonyl-2-pyrimidyl)phenol and p-(5-decyl-2-pyrimidyl)phenyl, respectively, in place of the p-(5-dodecyl-2-pyrimidyl)phenol to obtain the following compounds:

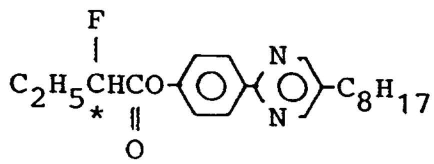

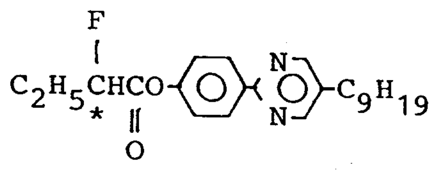

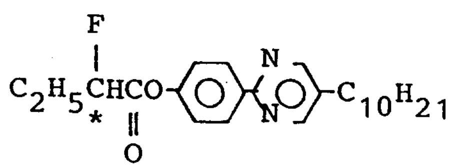

The phase transition temperature and optical rotation data for these compounds are shown in the following Table 2 together with those for the compounds of Examples 9 and 10. The symbols used in Table 2 have the same meanings as in Table 1.

Table 2

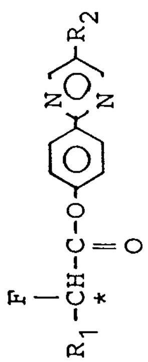

| Example | $R_1$ | $R_2$ | Optical rotation ($[\alpha]_D$) | Phase transition temperature (°C) |
|---|---|---|---|---|
| 9 | $C_2H_5$- | $-C_{12}H_{25}$ | -1.22° (27°C) (c=0.82, $CH_2Cl_2$) | Cryst. $\xrightarrow{69}$ Iso. $\xrightarrow{59}$ SmA $\xrightarrow{50}$ Cryst. |
| 10 | $CH_3$- | $-C_{10}H_{21}$ | - | Cryst. $\underset{53}{\overset{71}{\rightleftarrows}}$ Iso. |
| 11 | $C_2H_5$- | $-C_8H_{17}$ | - | Cryst. $\underset{48}{\overset{60}{\rightleftarrows}}$ Iso. |
| 12 | $C_2H_5$- | $-C_9H_{19}$ | +1.78° (32°C) (c-0.79, $Et_2O$) | Cryst. $\xrightarrow{70}$ Iso. $\xrightarrow{58}$ $S_3$ $\xrightarrow{55}$ Cryst. |
| 13 | $C_2H_5$- | $-C_{10}H_{21}$ | +2.86° (32°C) (c=0.98, $CH_2Cl_2$) | Cryst. $\underset{57}{\overset{69}{\rightleftarrows}}$ Iso. |

Example 14

A liquid crystal Composition C shown below was prepared by using the mesomorphic compound of Example 13 in Table 2. The composition, phase transition temperature and optical rotation are shown below in parallel with those of the Composition A in Example 8 which was different from the Composition C only in that the mesomorphic compound of Example 1 having a longer carbon chain in its optically active group was used instead of the Composition C.

<Liquid Crystal Composition C>

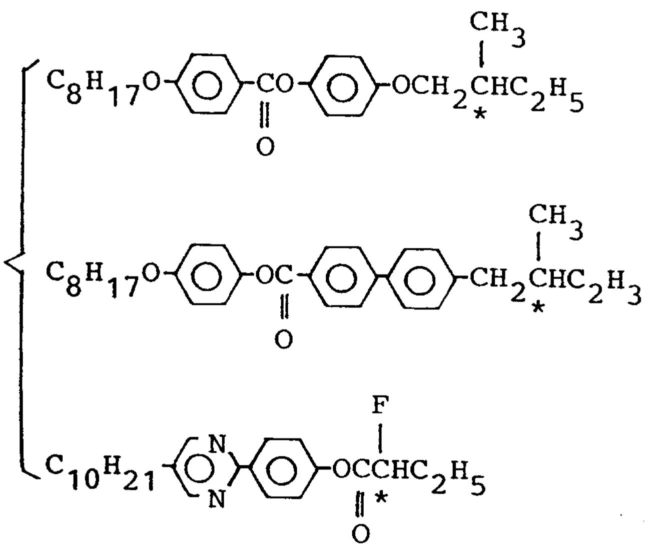

72.0 wt.%

18.0 wt.%

10.0 wt.%

Phase transition temperature (°C)

Cryst. $\underset{11}{\overset{12}{\rightleftarrows}}$ Sc* $\underset{42}{\overset{43}{\rightleftarrows}}$ $S_A$ $\underset{60}{\overset{61}{\rightleftarrows}}$ Ch. $\underset{70}{\overset{70}{\rightleftarrows}}$ Iso.

<Liquid Crystal Composition A>

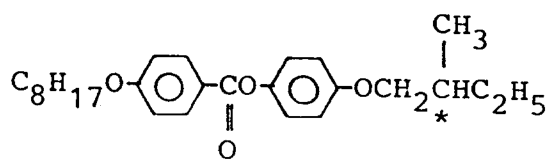

72.0 wt.%

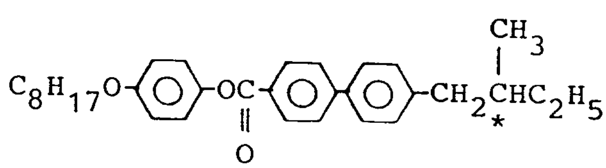

18.0 wt.%

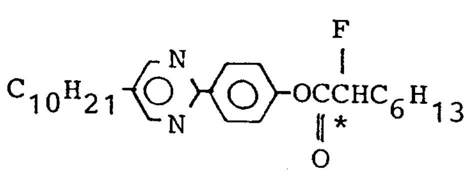

10.0 wt.%

Phase transition temperature (°C)

Cryst. $\underset{10}{\overset{17}{\rightleftarrows}}$ Sc* $\underset{44}{\overset{45}{\rightleftarrows}}$ $S_A$ $\underset{60}{\overset{61}{\rightleftarrows}}$ Ch. $\underset{70}{\overset{70}{\rightleftarrows}}$ Iso.

Spontaneous polarization (nC/cm$^2$)

| Temp. (°C) | L.C. Composition C | L.C. Composition A |
|---|---|---|
| 40 | 3.6 | 4.3 |
| 30 | 8.1 | 7.7 |

A ferroelectric liquid crystal device was prepared in the same manner as in Example 8 by using the above Liquid Crystal Composition C.

The optical response time of the ferroelectric liquid crystal device thus produced was measured similarly and is shown below together with that of the device using the Composition A.

Optical response time (msec)

| Temp. (°C) | L.C. Composition C | L.C. Composition A |
|---|---|---|
| 40 | 0.03 | 0.06 |
| 30 | 0.08 | 0.13 |

The above results show that the liquid crystal Compositions C and A did not show substantial difference with respect to phase transition temperature or spontaneous polarization, but the Composition C containing a mesomorphic compound having a shorter carbon chain in the optically active group showed a higher response speed.

Example 15

Two glass plates each provided with an ITO transparent electrode film were coated with a film of a polyimide resin precursor by spinner coating, followed by heating at 300°C for 60 minutes to form polyimide films. The polyimide films were further treated by rubbing, and the two glass plates thus treated were applied to each other to form a blank cell having a cell gap of 8 µm. The cell was filled with a biphenyl-type nematic liquid crystal composition to form a TN-type cell. The cell was then observed through a polarizing microscope, whereby a reverse domain (fringe pattern) was observed.

A TN-type cell was prepared similarly except for using a liquid crystal composition obtained by adding 1 wt. part of the mesomorphic compound of Example 2 above to 99 wt. parts of the above-mentioned biphenyl-type nematic liquid crystal composition. As a result of observation through a polarizing microscope, no reverse domain was observed but a uniform nematic phase was found to be formed. Thus, it was found that the mesomorphic compound according to the present invention was effective in preventing occurrence of reverse domain.

As described above, according to the present invention, there is provided a mesomorphic compound represented by the above-mentioned formula (I) and having a fluorine atom providing a large dipole moment directly bonded to its asymmetric carbon atom. Further, by adding at least one species of the mesomorphic compound as a constituent, it is possible to provide a TN-type liquid crystal composition with little occurrence of reverse domain, or a chiral nematic liquid crystal composition or a chiral smectic liquid crystal composition with an improved electric field-responsive characteristic. It is further possible to control the liquid crystal state of the resultant liquid crystal composition.

## Claims

1. An optically active mesomorphic compound represented by the following formula (I):

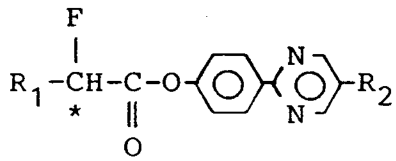

(I),

wherein $R_1$ and $R_2$ are respectively an alkyl group having 1 to 16 carbon atoms, and C* denotes an asymmetric carbon atom.

2. A compound according to Claim 1, which is

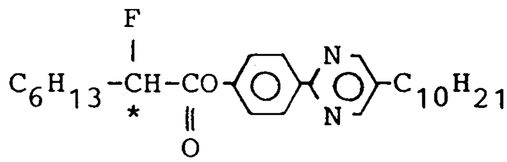

.

3. A compound according to Claim 1, which is

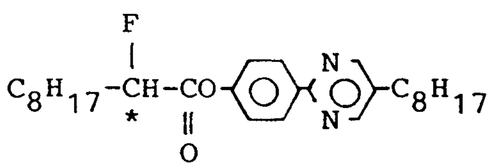

.

4. A compound according to Claim 1, which is

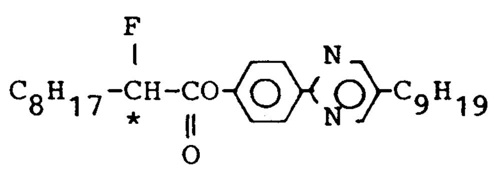

.

5. A compound according to Claim 1, which is

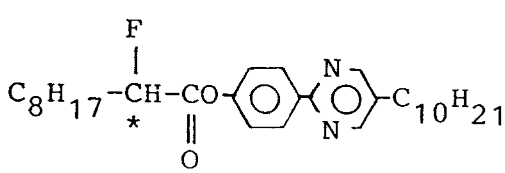

.

**6.** A compound according to Claim 1, which is

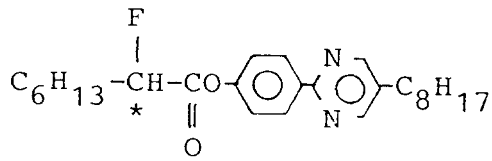

.

**7.** A compound according to Claim 1, which is

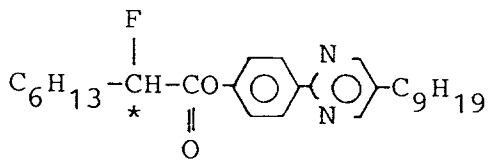

.

**8.** A compound according to Claim 1, which is

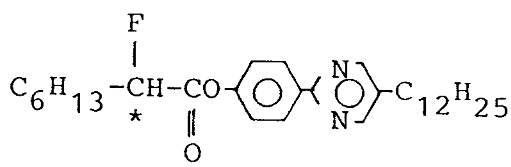

.

**9.** A compound according to Claim 1, which is

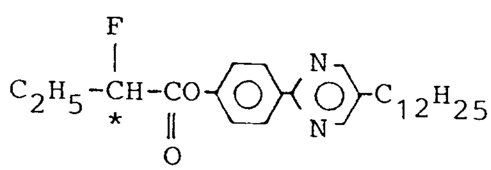

.

**10.** A compound according to Claim 1, which is

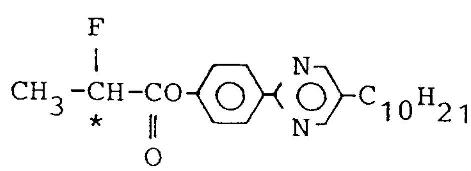

.

**11.** A compound according to Claim 1, which is

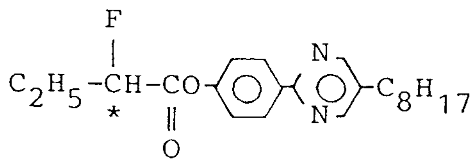

.

**12.** A compound according to Claim 1, which is

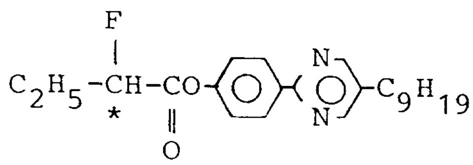

.

**13.** A compound according to Claim 1, which is

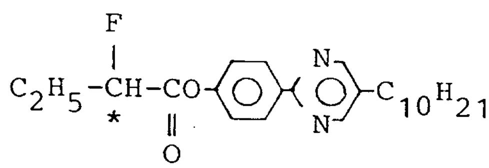

.

**14.** A liquid crystal composition, consisting of at least two compounds and containing at least one compound represented by the following formula (I):

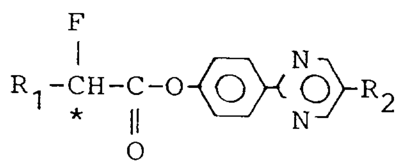

(I),

wherein $R_1$ and $R_2$ are respectively an alkyl group having 1 to 16 carbon atoms, and C* denotes an asymmetric carbon atom.

**15.** A liquid crystal composition according to Claim 14, which contains a ferroelectric liquid crystal in addition to said at least one compound, said ferroelectric liquid crystal being selected from the group of

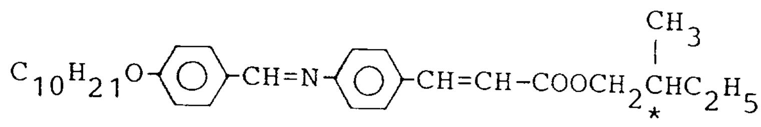

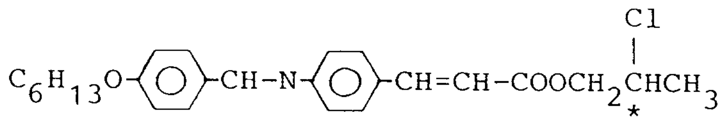

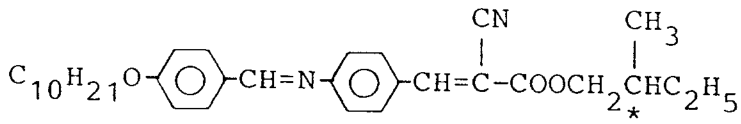

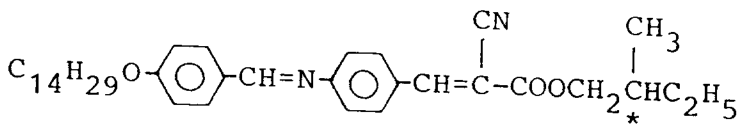

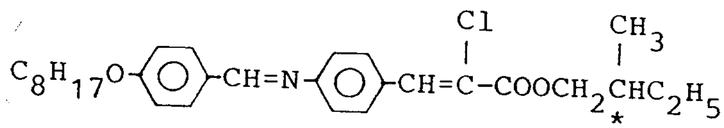

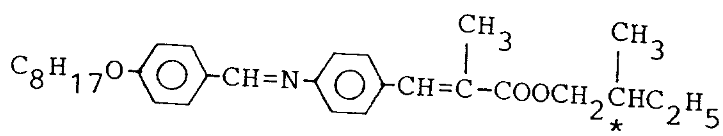

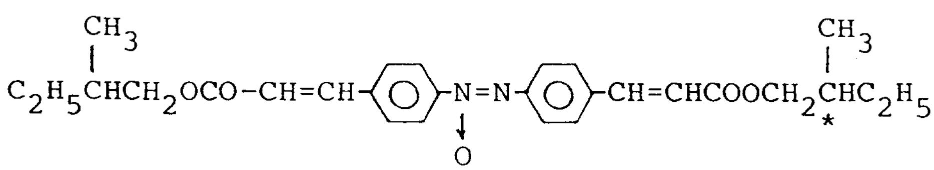

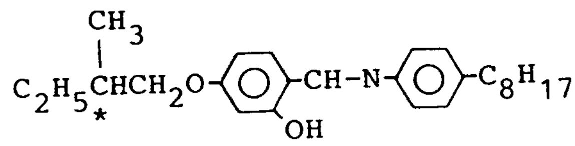

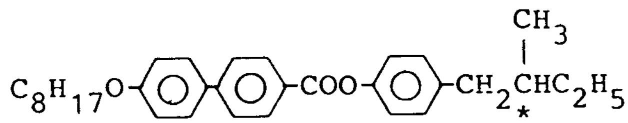

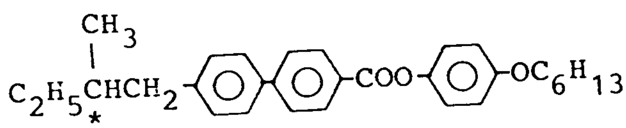

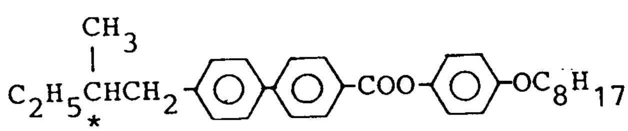

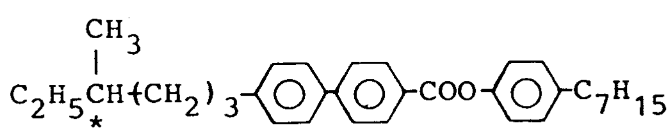

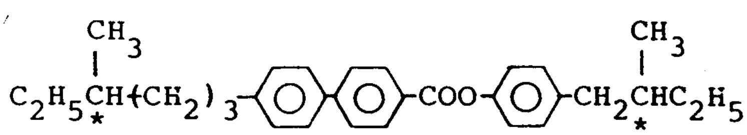

16. A liquid crystal composition according to Claim 14, which contains a non-chiral smectic liquid crystal in addition to at least one said compound.

17. A liquid crystal composition according to Claim 14, which contains a nematic liquid crystal in addition to at least one said compound.

18. A liquid crystal device, which comprises a pair of oppositely spaced electrodes and a liquid crystal composition disposed between the oppositely spaced electrodes; said liquid crystal composition consisting of at least two compounds and containing at least one compound represented by the following formula (I):

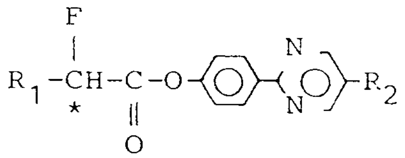

(I),

wherein $R_1$ and $R_2$ are respectively an alkyl group having 1 to 16 carbon atoms, and C* denotes an asymmetric carbon atom.

**19.** A liquid crystal device according to Claim 18, wherein said liquid crystal composition contains a ferroelectric liquid crystal in addition to at least one said compound, said ferroelectric liquid crystal being selected from the group of

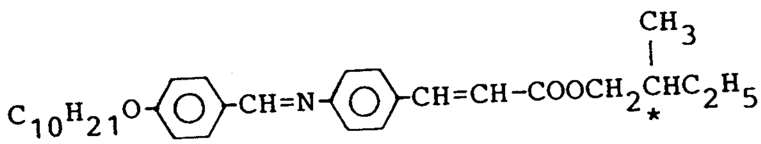

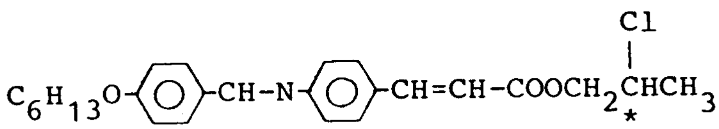

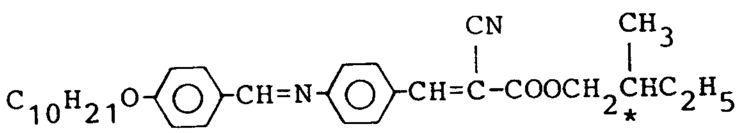

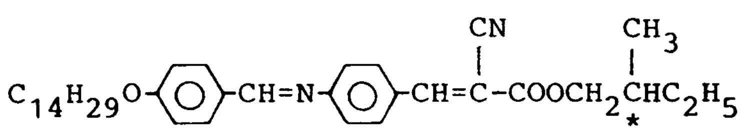

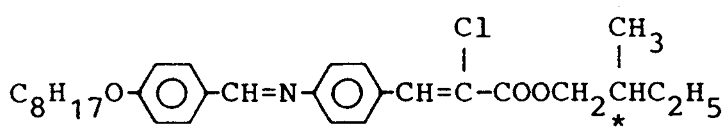

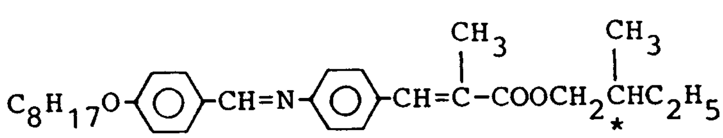

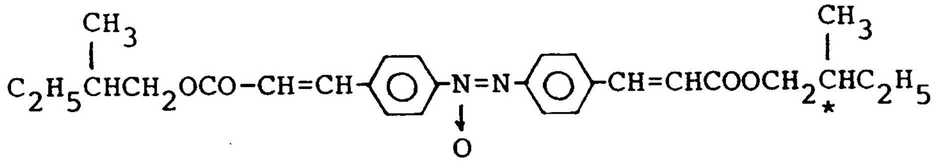

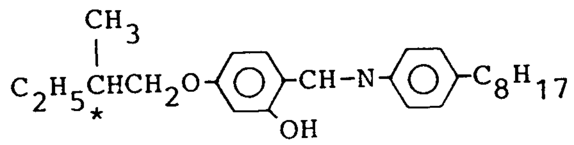

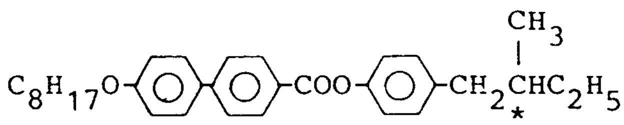

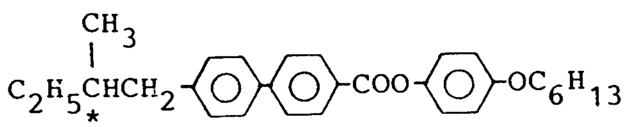

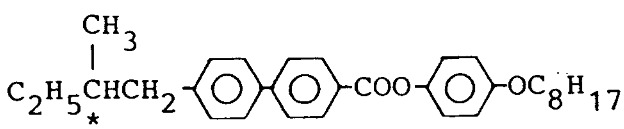

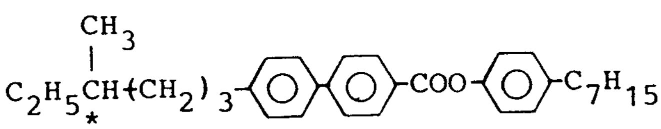

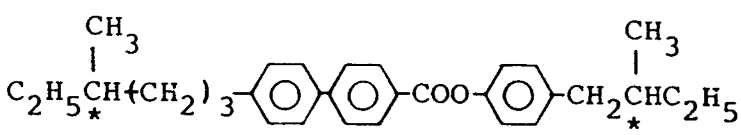

**20.** A liquid crystal device according to Claim 18, wherein said liquid crystal composition contains a non-chiral smectic liquid crystal in addition to at least one said compound.

**21.** A liquid crystal device according to Claim 18, wherein said liquid crystal composition contains a nematic liquid cystal in addition to at least one said compound.

## Patentansprüche

1. Optisch aktive mesomorphe Verbindung, die durch die folgende Formel (I) dargestellt wird:

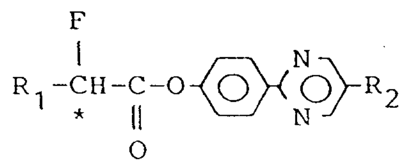

(I)

worin $R_1$ und $R_2$ jeweils eine Alkylgruppe mit 1 bis 16 Kohlenstoffatomen bedeuten und C* ein asymmetrisches Kohlenstoffatom bezeichnet.

2. Verbindung nach Anspruch 1, die

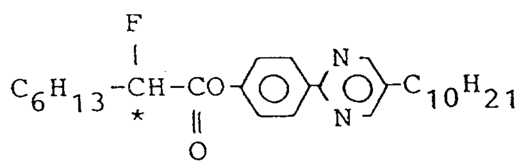

ist.

3. Verbindung nach Anspruch 1, die

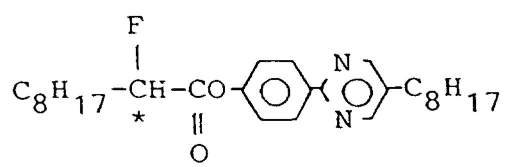

ist.

4. Verbindung nach Anspruch 1, die

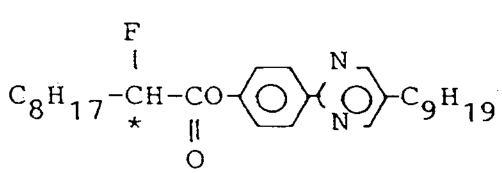

ist.

5. Verbindung nach Anspruch 1, die

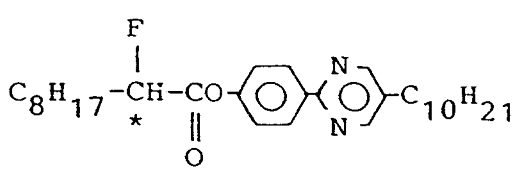

ist.

**6.** Verbindung nach Anspruch 1, die

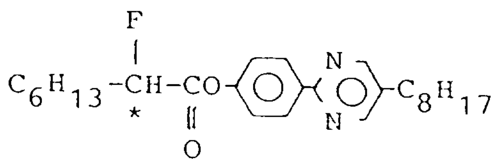

ist.

**7.** Verbindung nach Anspruch 1, die

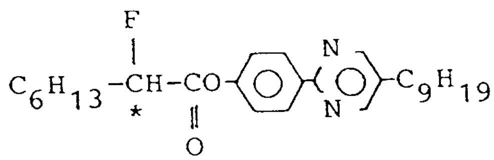

ist.

**8.** Verbindung nach Anspruch 1, die

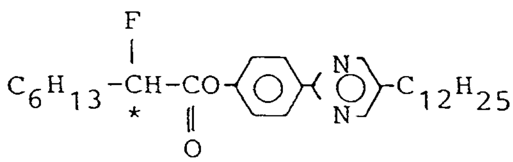

ist.

**9.** Verbindung nach Anspruch 1, die

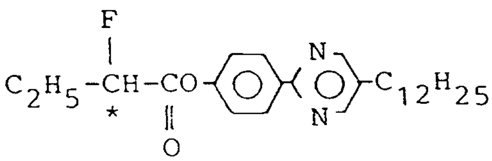

ist.

**10.** Verbindung nach Anspruch 1, die

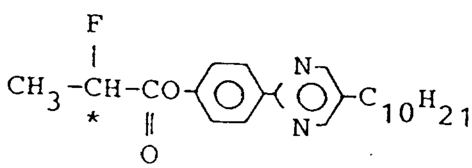

ist.

**11.** Verbindung nach Anspruch 1, die

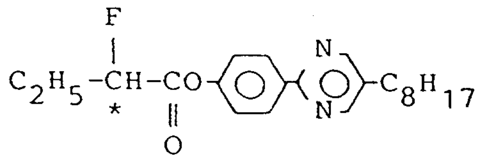

ist.

**12.** Verbindung nach Anspruch 1, die

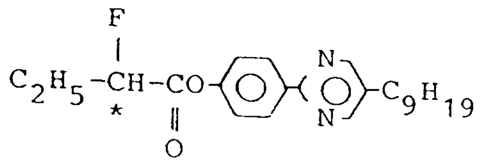

ist.

**13.** Verbindung nach Anspruch 1, die

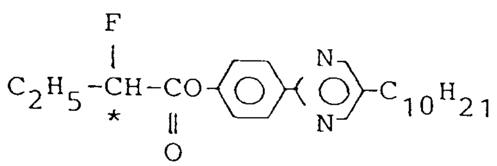

ist.

**14.** Flüssigkristallmischung, die aus mindestens zwei Verbindungen besteht und mindestens eine Verbindung enthält, die durch die folgende Formel (I) dargestellt wird:

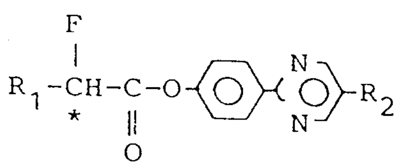

(I)

worin $R_1$ und $R_2$ jeweils eine Alkylgruppe mit 1 bis 16 Kohlenstoffatomen bedeuten und C* ein asymmetrisches Kohlenstoffatom bezeichnet.

**15.** Flüssigkristallmischung nach Anspruch 14, die zusätzlich zu der erwähnten mindestens einen Verbindung einen ferroelektrischen Flüssigkristall enthält, wobei der erwähnte ferroelektrische Flüssigkristall aus der folgenden Gruppe ausgewählt ist:

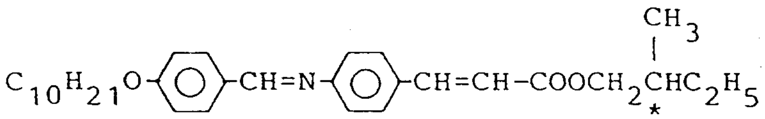

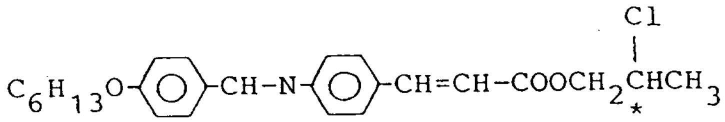

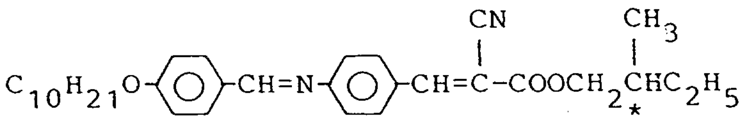

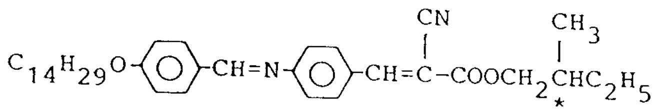

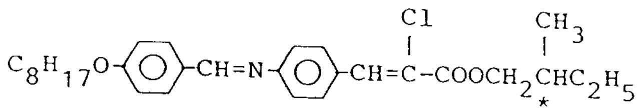

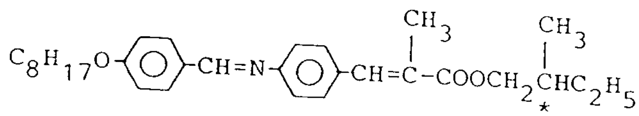

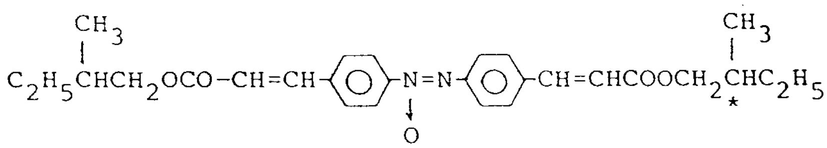

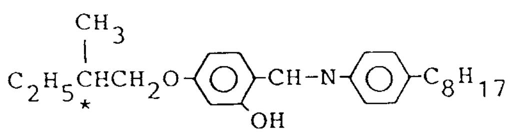

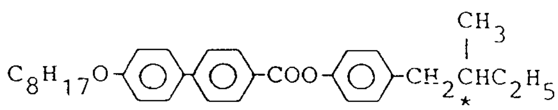

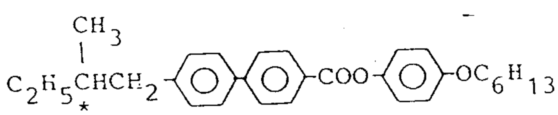

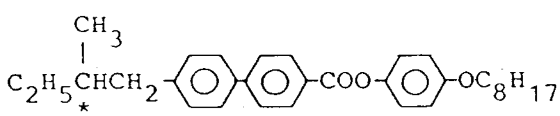

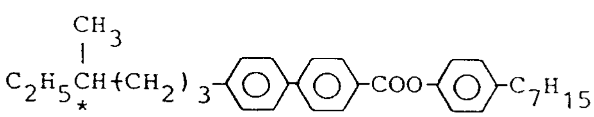

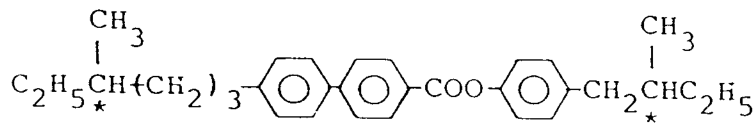

16. Flüssigkristallmischung nach Anspruch 14, die zusätzlich zu mindestens einer erwähnten Verbindung einen nichtchiralen smektischen Flüssigkristall enthält.

17. Flüssigkristallmischung nach Anspruch 14, die zusätzlich zu mindestens einer erwähnten Verbindung einen nematischen Flüssigkristall enthält.

18. Flüssigkristallvorrichtung mit einem Paar Elektroden, die mit Abstand gegenüberliegend angeordnet sind, und einer Flüssigkristallmischung, die zwischen den mit Abstand gegenüberliegend angeordneten Elektroden angeordnet ist, wobei die erwähnte Flüssigkristallmischung aus mindestens zwei Verbindungen besteht und mindestens eine Verbindung enthält, die durch die folgende Formel (I) dargestellt wird:

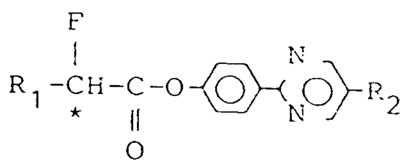

(I)

worin $R_1$ und $R_2$ jeweils eine Alkylgruppe mit 1 bis 16 Kohlenstoffatomen bedeuten und C* ein asymmetrisches Kohlenstoffatom bezeichnet.

19. Flüssigkristallvorrichtung nach Anspruch 18, bei der die erwähnte Flüssigkristallmischung zusätzlich zu mindestens einer erwähnten Verbindung einen ferroelektrischen Flüssigkristall enthält, wobei der erwähnte ferroelektrische Flüssigkristall aus der folgenden Gruppe ausgewählt ist:

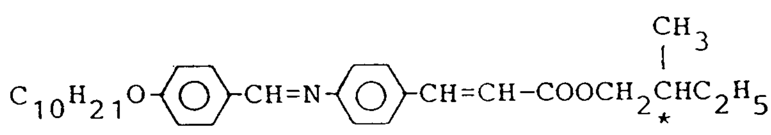

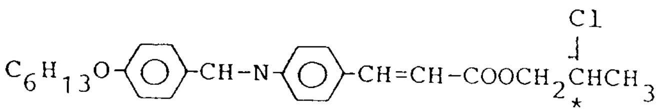

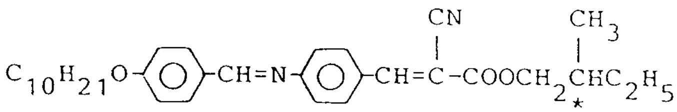

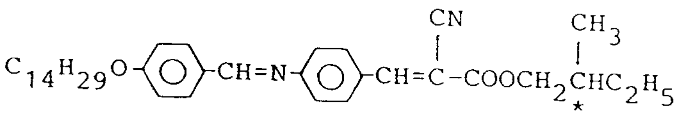

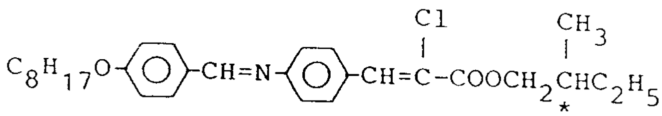

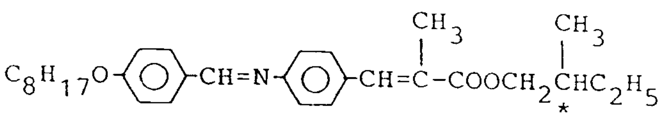

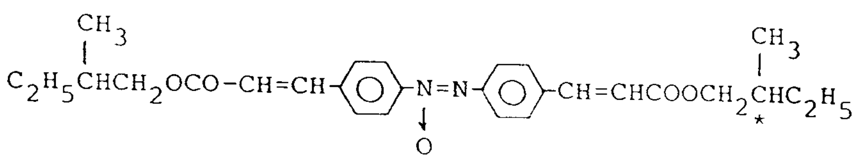

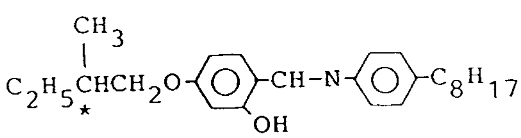

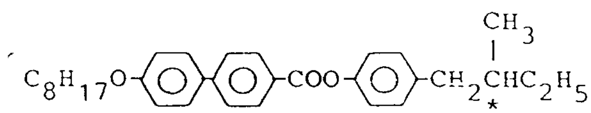

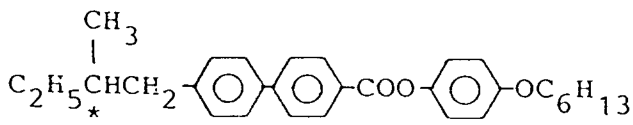

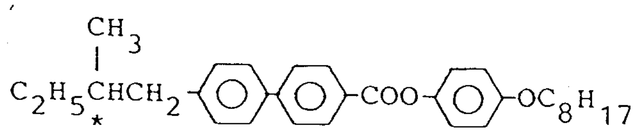

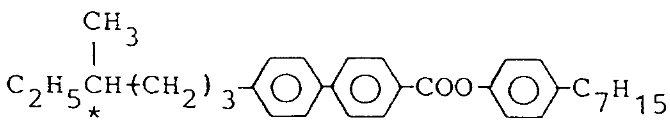

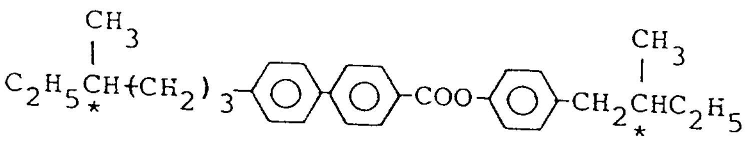

**20.** Flüssigkristallvorrichtung nach Anspruch 18, bei der die erwähnte Flüssigkristallmischung zusätzlich zu mindestens einer erwähnten Verbindung einen nichtchiralen smektischen Flüssigkristall enthält.

**21.** Flüssigkristallvorrichtung nach Anspruch 18, bei der die erwähnte Flüssigkristallmischung zusätzlich zu mindestens einer erwähnten Verbindung einen nematischen Flüssigkristall enthält.

## Revendications

**1.** Composé mésomorphe optiquement actif représenté par la formule (I) suivante :

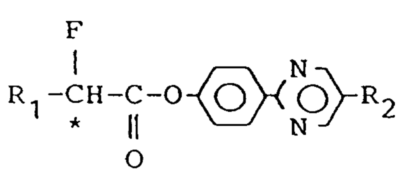

(I),

dans laquelle $R_1$ et $R_2$ représentent respectivement un groupe alkyle ayant 1 à 16 atomes de carbone et C* désigne un atome asymétrique de carbone.

**2.** Composé suivant la revendication 1, qui est le composé

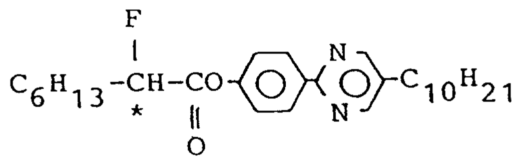

.

**3.** Composé suivant la revendication 1, qui est le composé

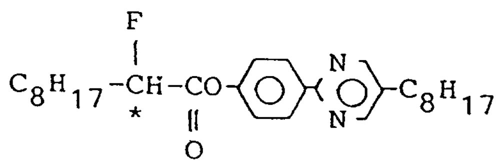

.

**4.** Composé suivant la revendication 1, qui est le composé

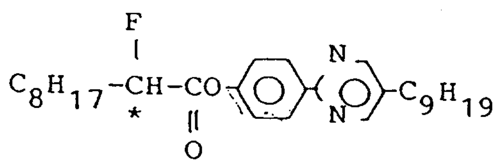

.

**5.** Composé suivant la revendication 1, qui est le composé

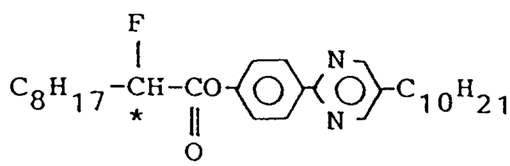

.

**6.** Composé suivant la revendication 1, qui est le composé

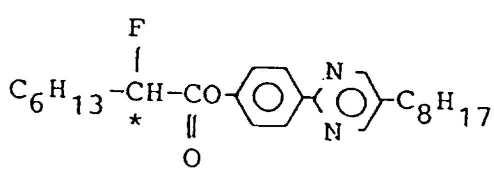

.

**7.** Composé suivant la revendication 1, qui est le composé

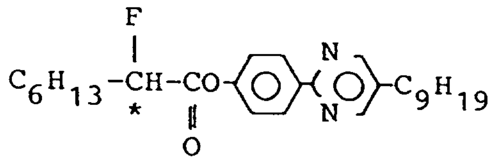

.

**8.** Composé suivant la revendication 1, qui est le composé

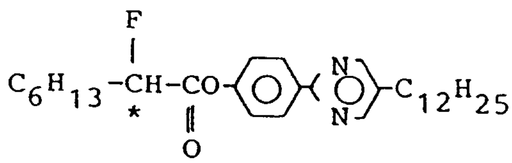

.

**9.** Composé suivant la revendication 1, qui est le composé

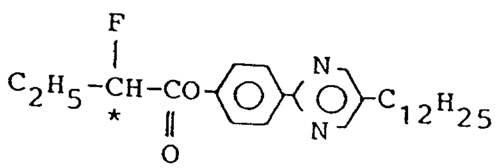

.

**10.** Composé suivant la revendication 1, qui est le composé

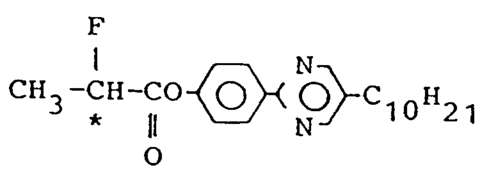

.

**11.** Composé suivant la revendication 1, qui est le composé

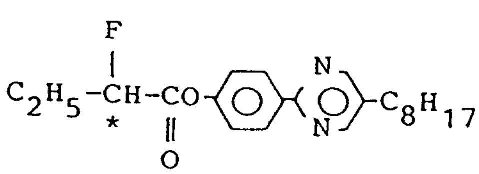

.

**12.** Composé suivant la revendication 1, qui est le composé

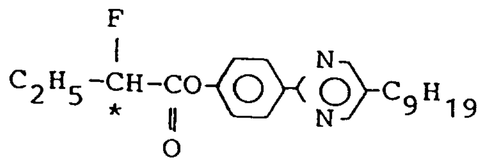

.

**13.** Composé suivant la revendication 1, qui est le composé

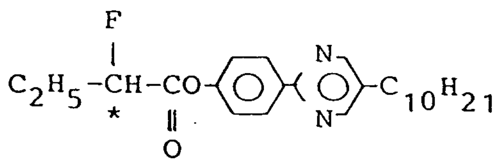

.

**14.** Composition à cristaux liquides, constituée d'au moins deux composés et contenant au moins un composé représenté par la formule (I) suivante :

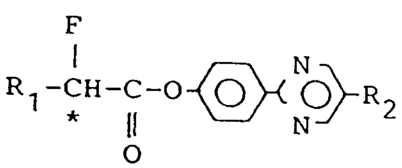

(I),

dans laquelle $R_1$ et $R_2$ représentent respectivement un groupe alkyle ayant 1 à 16 atomes de carbone et C* désigne un atome asymétrique de carbone.

**15.** Composition à cristaux liquides suivant la revendication 14, qui contient un cristal liquide ferroélectrique en plus du composé ayant au moins un représentant, ce cristal liquide ferro-électrique étant choisi dans le groupe des composés

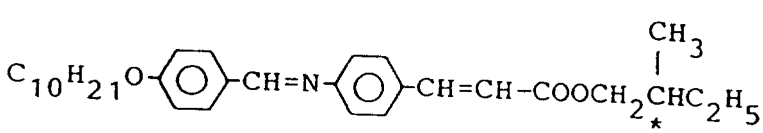

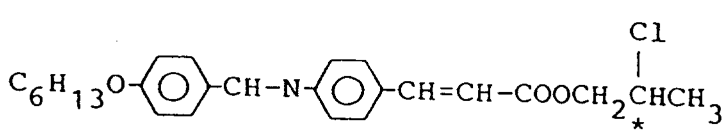

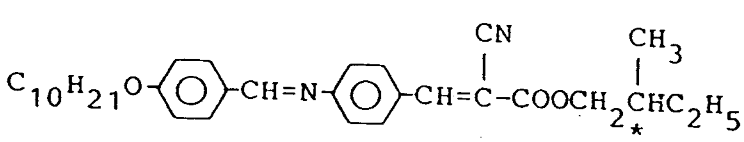

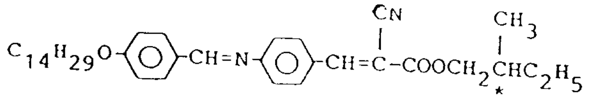

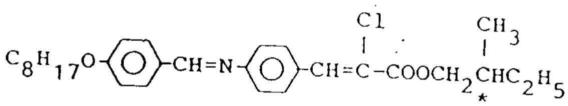

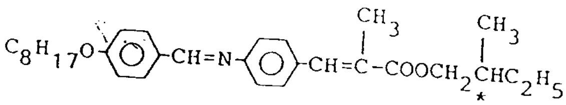

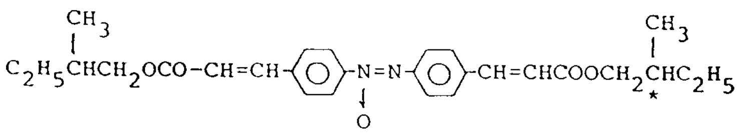

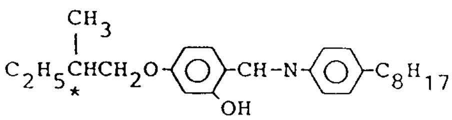

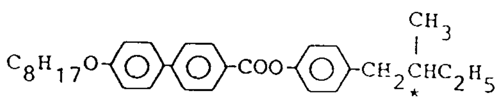

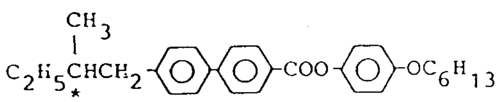

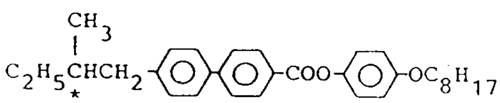

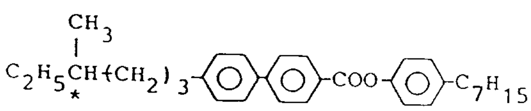

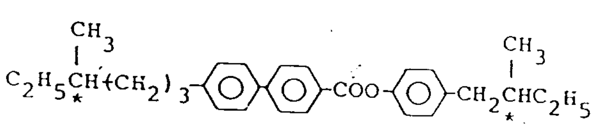

**16.** Composition à cristaux liquides suivant la revendication 14, qui contient un cristal liquide smectique non chiral en plus du composé ayant au moins un représentant.

**17.** Composition à cristaux liquides suivant la revendication 14, qui contient un cristal liquide nématique en plus du composé ayant au moins un représentant.

**18.** Dispositif à cristaux liquides, qui comprend une paire d'électrodes espacées l'une vis-à-vis de l'autre et une composition à cristaux liquides disposée entre les électrodes espacées l'une en face de l'autre ; ladite composition à cristaux liquides étant constituée d'au moins deux composés et contenant au moins un composé représenté par la formule (I) suivante :

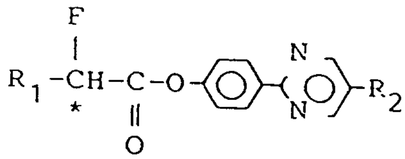

(I),

dans laquelle $R_1$ et $R_2$ représentent respectivement un groupe alkyle ayant 1 à 16 atomes de carbone et C* désigne un atome asymétrique de carbone.

**19.** Dispositif à cristaux liquides suivant la revendication 18, dans lequel la composition à cristaux liquides contient un cristal liquide ferro-électrique en plus du composé dont il existe au moins un représentant, ledit cristal liquide ferro-électrique étant choisi dans le groupe des composés

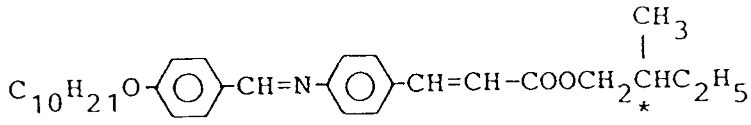

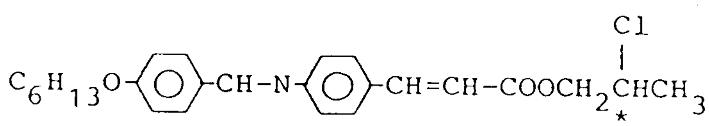

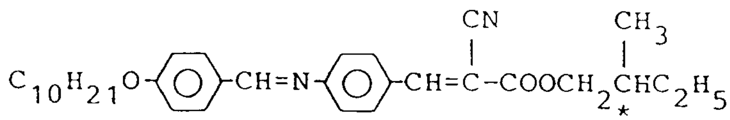

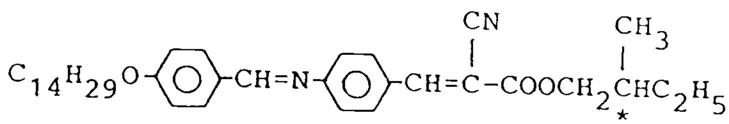

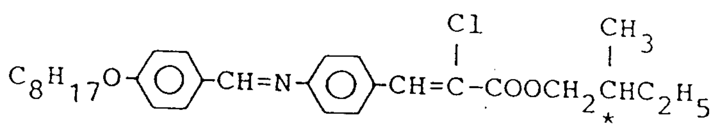

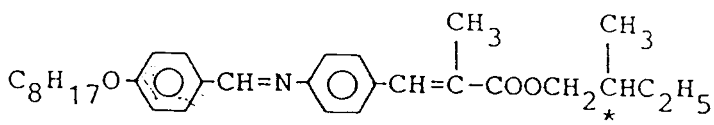

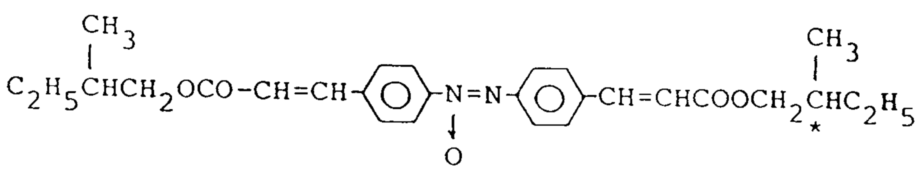

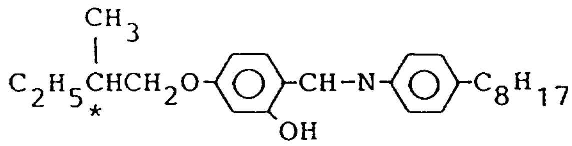

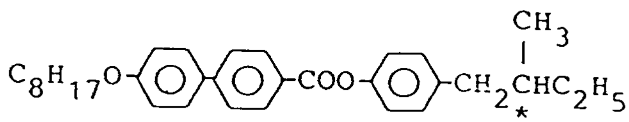

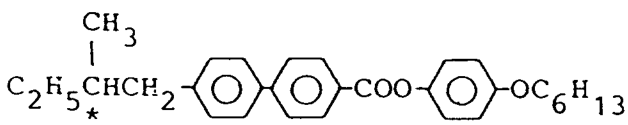

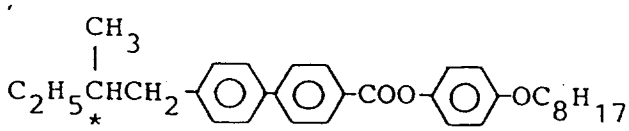

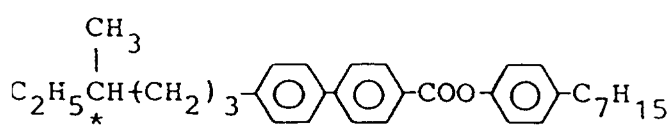

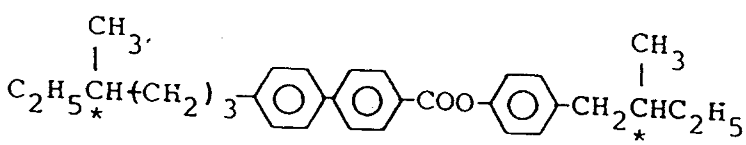

**20.** Dispositif à cristaux liquides suivant la revendication 18, dans lequel la composition à cristaux liquides contient un cristal liquide smectique non chiral en plus du composé dont il existe au moins un représentant.

**21.** Dispositif à cristaux liquides suivant la revendication 18, dans lequel la composition à cristaux liquides contient un cristal liquide nématique en plus du composé dont il existe au moins un représentant.